# EUROPEAN PATENT SPECIFICATION

(11) **EP 3 225 233 B1**
(45) Date of publication and mention of the grant of the patent: **14.08.2019**
(21) Application number: 17166616.7
(22) Date of filing: 27.10.2006
(51) Int. Cl.: A61K 9/08, A61K 9/19, A61K 47/02, A61K 47/18, A61K 47/26, A61K 38/48

(54) **SODIUM CHLORIDE SOLUTION FOR DRUG RECONSTITUTION**
NATRIUMCHLORIDLÖSUNG FÜR DIE ARZNEIMITTEL-REKONSTITUTION
SOLUTION DE CHLORURE DE SODIUM POUR LA RECONSTITUTION DE MEDICAMENTS

(30) Priority: 01.11.2005 US 732221 P
(43) Date of publication of application: 04.10.2017
(62) Divisional of application: 06827036.2
(73) Proprietor: Wyeth LLC, New York, NY 10017-5755 (US)
(72) Inventor: WEBB, Chandra A., Pelham, NH 03076 (US); ZERFAS, Julie, Reading, MA 01867 (US)
(74) Representative: Pfizer

(56) References cited:
- WO-A-2005/058283
- WO-A-2005/089712
- US-A- 3 717 708
- US-A1- 2001 031 721
- VAN DEN BERG H M ET AL: "BeneFixTM induces agglutination of red cells", BLOOD, W.B.SAUNDERS COMPANY, ORLANDO, FL, US, vol. 96, no. 11 Part 1, 16 November 2000 (2000-11-16), page 641a, XP008083763, ISSN: 0006-4971
- BUSH L ET AL: "THE FORMULATION OF RECOMBINANT FACTOR IX: STABILITY, ROBUSTNESS, AND CONVENIENCE", SEMINARS IN HEMATOLOGY, PHILADELPHIA, PA, US, vol. 35, no. 2, SUPPL 2, April 1998 (1998-04), pages 18-21, XP001010494, ISSN: 0037-1963

## Description

A portion of the disclosure of this patent document contains material that is subject to copyright protection. The copyright owner has no objection to the facsimile reproduction by anyone of the patent document or the patent disclosure, as it appears in the Patent and Trademark Office patent file or records, but otherwise reserves all copyright rights whatsoever.

### BACKGROUND OF THE INVENTION

When whole blood is mixed with a drug solution, such as in the intravenous (IV) line during administration of an IV injectable drug, rouleaux or erythrocyte aggregation (also called red blood cell agglutination) may occur if the drug solution does not contain sufficient ionic strength. Erythrocyte aggregation occurs, for example, with 5% dextrose in water (a common large volume parenteral solution) and with many pharmaceutical products that have low ionic strength.

Pharmaceutical products are often lyophilized, and therefore need to be reconstituted with a solution prior to parenteral injection. However, when lyophilized products are reconstituted with low ionic strength solutions, the resultant preparation can also cause erythrocyte aggregation. Although reconstitution of lyophilized cakes with normal saline solution (0.9% NaCl) as opposed to sterile water for injection (sWFI) would provide additional ionic strength, the resulting drug solution may be hypertonic and could have undesirable side effects upon injection.

Van Den Berg et al., Blood vol. 96, no.11 Part 1 relates to agglutination of red blood cells induced by BeneFix™. WO2005/089712 Al relates to a lyophilisation method for pharmaceutical formulations, including Factor IX formulations, to improve excipient crystallisation.

Thus, there remains a need for a solution that can be used to reconstitute lyophilized cakes or to dilute pharmaceutical solutions to provide a resultant preparation for injection that is isotonic with respect to plasma and has sufficient ionic strength such that it does not cause erythrocyte aggregation.

### SUMMARY OF THE INVENTION

The invention is based on the discovery that erythrocyte agglutination is caused by low ionic strength solutions that come into contact with blood. Thus, when pharmaceutical formulations are prepared for intravenous injection by reconstitution or dilution in low ionic strength solutions such as 5% dextrose, 3% dextran, or sWFI, the resultant preparations may have the requisite osmolarity to be about isotonic with respect to blood, but they often do not have an ionic strength sufficient to prevent agglutination. On the other hand, when pharmaceutical formulations are prepared for intravenous injection by reconstitution or dilution with high ionic strength solutions such as saline (0.9% NaCl or 154 mM NaCl), the resultant preparation may have a sufficient ionic strength to prevent agglutination, but it may possess an osmolarity that is hypertonic with respect to blood, thereby causing dehydration of red blood cells (RBCs), venous inflammation, and/or possibly thrombophlebitis if repeated injections are frequent or chronic.

The invention therefore provides a lyophilized Factor IX formulation and a 25 mM to 150 mM sodium chloride solution for use in a method for preparing a lyophilized Factor IX formulation for intravenous injection wherein the method comprises adding the sodium chloride solution to the lyophilized formulation thereby resulting in a formulation prepared for intravenous injection, wherein the prepared formulation is about isotonic with respect to plasma and has an osmolarity that is from 270 mOsm/L to 330 mOsm/L, or,
is slightly hypotonic with respect to plasma and has an osmolarity that is 220 mOsm/L to 270 mOsm/L, or,
is slightly hypertonic with respect to plasma and has an osmolarity that is 330 mOsm/L to 600 mOsm/L,
and wherein the prepared formulation has a sufficient ionic strength to prevent erythrocyte agglutination upon intravenous injection,
wherein the lyophilized formulation does not contain more than 25mM of an ionizing salt when it is reconstituted in water in a volume that is the same as the fill volume.

The invention also provides a kit comprising the lyophilized factor IX formulation and sodium chloride solution referred to above.

The prepared formulation is about isotonic with respect to plasma, for example, when it has an osmolarity that is from 270 mOsm/L to 330 mOsm/L. The prepared formulation is slightly hypotonic with respect to plasma, for example, when it has an osmolarity that is from 220 mOsm/L to 270 mOsm/L. The prepared formulation is slightly hypertonic with respect to plasma, for example, when it has an osmolarity that is from 330 mOsm/L to 600 mOsm/L.

The prepared formulation has an ionic strength that is sufficient to prevent erythrocyte agglutination when it has at least 25 mEq/L of Na⁺ and Cl⁻ ions. In another aspect, the prepared formulation has an ionic strength that is sufficient to prevent erythrocyte agglutination when it has, for example, at least 40 mEq/L of Na⁺ and Cl⁻ ions. In another aspect, the prepared formulation has an ionic strength that is sufficient to prevent erythrocyte agglutination when it has, for example, at least 40 mEq/L of Na⁺ and Cl⁻ ions and less than 150 mEq/L of Na⁺ and Cl⁻ ions. In another aspect, the prepared formulation has an ionic strength that is sufficient to prevent erythrocyte agglutination when it has, for example, an ionic strength as measured in conductivity that is at least about 2.5 mS/cm. In another aspect, the prepared formulation has an ionic strength that is sufficient to prevent erythrocyte agglutination when it has, for example, an ionic strength as measured in conductivity that is at least about 4.0 mS/cm.

The pharmaceutical formulation to be prepared for injection is a lyophilized formulation. The formulation can therefore be reconstituted into solution by a sodium chloride solution that has a sodium chloride concentration of 25-150 mM, 25-100 mM, 25-80 mM, 25-40 mM, 25-35 mM, 25-30 mM, or about 40 mM.

In one aspect, the sodium chloride solution that is added comprises from 40 mM to 150 mM sodium chloride. In one aspect, the sodium chloride solution that is added consists essentially of from 40 mM to 150 mM sodium chloride. In one aspect, the sodium chloride solution that is added comprises about 40 mM sodium chloride. In one aspect, the sodium chloride solution that is added consists essentially of a 40 mM sodium chloride solution. In one aspect, the sodium chloride solution that is added consists essentially of a solution that has a sodium chloride solution that is about 40 mM ± 10 mM sodium chloride.

In one aspect, wherein prior to the addition of the sodium chloride solution, the pharmaceutical formulation does not contain an appreciable amount of an ionizing salt. An appreciable amount of an ionizing salt can be, for example, an amount that is greater than about 5 mM. In another aspect, an appreciable amount of an ionizing salt can be, for example, an amount that is greater than about 25 mM. If the pharmaceutical formulation is a lyophilized formulation, it does not contain an appreciable amount of an ionizing salt if it does not contain more than, for example, 5 mM or 25 mM of an ionizing salt when the lyophilized formulation is reconstituted in water.

In one aspect, wherein prior to the addition of the sodium chloride solution, the pharmaceutical formulation comprises histidine, glycine, sucrose, and polysorbate. In another aspect, wherein prior to the addition of the sodium chloride solution, the pharmaceutical formulation comprises histidine, glycine, sucrose, polysorbate, and a therapeutic protein, which is Factor IX. In another aspect, wherein prior to the addition of the sodium chloride solution, the pharmaceutical formulation comprises histidine, glycine, sucrose, polysorbate, and Factor IX (including recombinant Factor IX (rFIX)). As used herein, Factor IX can include modified versions of Factor IX, including for example, PEGylated Factor IX, protein fusions comprising Factor IX such as albumin-Factor IX or immunoglobulin (whole or domains thereof)-Factor IX, and glycosylated Factor IX.

In one aspect, wherein the pharmaceutical formulation is a lyophilized formulation, prior to the addition of the sodium chloride solution, the formulation, measured as if it was reconstituted in water (in a volume that is the same as the fill volume, *i.e.,* the volume of the formulation prior to lyophilization), comprises: (a) from about 5 mM to about 30 mM histidine; (b) from about 0.1M to about 0.3M glycine; (c) from about 0.5 to about 2 percent sucrose; and (d) from about 0.001 to about 0.05 percent polysorbate (or from about 0.005 to about 0.05 percent). In one aspect, the formulation can further comprise, measured if it was reconstituted in water, (e) from about 0.1 mg/mL to about 100 mg/mL or more of a therapeutic protein, or from about 10, 50, 100, 200, 300, 400, 500, 1000, 2000 IU/mL (international units/mL) or more of a therapeutic protein. In one aspect, the formulation can further comprise, measured if it was reconstituted in water, (e) from about 0.1 mg/mL to about 100 mg/mL or more of Factor IX, or from about 0.4 mg/mL to about 20 mg/mL of Factor IX, or from about 10, 50, 100, 200, 300, 400, 500, 1000, 2000 IU/mL (international units/mL) or more of Factor IX.

The lyophilized factor IX formulation and sodium chloride solution of the invention can be used in a method for preventing erythrocyte agglutination caused from intravenous injection, the method comprising reconstituting or diluting a pharmaceutical formulation with a 25 mM to 150 mM sodium chloride solution such that the reconstituted or diluted pharmaceutical formulation has an ionic strength sufficient to prevent erythrocyte agglutination when the reconstituted or diluted pharmaceutical formulation is administered to a subject by intravenous injection.

The lyophilized factor IX formulation and sodium chloride solution of the invention can be used in a method for preparing a lyophilized pharmaceutical formulation for intravenous injection, the method comprising reconstituting the lyophilized pharmaceutical formulation with a 25 mM to a 150 mM sodium chloride solution such that after reconstitution, the formulation has an ionic strength sufficient to prevent erythrocyte agglutination and an osmolarity that is about isotonic (or slightly hypertonic or slightly hypotonic).

In one aspect, the lyophilized formulation is reconstituted with the sodium chloride solution, wherein the volume of the sodium chloride solution used for reconstitution is less than the volume of the formulation pre-lyophilization (*i.e.,* fill volume). In this manner, the invention provides a method for reducing the volume of the formulation to be injected.

In one aspect, the lyophilized formulation is reconstituted with the sodium chloride solution, wherein the volume of the sodium chloride solution used for reconstitution is greater than the volume of the formulation pre-lyophilization (*i.e.,* fill volume). In this manner, the invention provides a method for maintaining the isotonicity of the formulation to be injected.

For example, a lyophilized formulation can be reconstituted with a volume of sodium chloride solution that is greater than the volume of the formulation pre-lyophilization, such as reconstitution with 5 mL of sodium chloride where the formulation volume pre-lyophilization is 4 mL. For example, a lyophilized 4 mL formulation reconstituted in 4 mL water is an isotonic solution containing 10 mM histidine, 260 mM glycine, 1% sucrose, 0.005% polysorbate, which is about 300 mOsm/L. But if the lyophilized formulation is reconstituted with 4 mL of a 40 mM NaCl (80 mOsm/L) solution, then the resultant formulation would have a slightly hypertonic solution (300 mOsm/L + 80 mOsm/L = 380 mOsm/L). But if the lyophilized formulation is reconstituted with 5 mL of a 40 mM NaCl solution, then the resulting solution is about 8 mM (8 mOsm/L) histidine, 208 mM (208 mOsm/L) glycine, 0.8% (24 mOsm/L) sucrose, 0.004% (negligible osmolarity) polysorbate, and 40 mM (80 mOsm/L) NaCl, which is about 320 mOsm/L. Thus, by reconstituting a pre-lyophilization formulation that is about isotonic with a volume of sodium chloride solution that is greater than the fill volume, the invention can provide a resulting solution that is still about isotonic. In other words, reconstituting a pre-lyophilization formulation that is about isotonic with a volume of sodium chloride solution that is less than or about the same as the fill volume can result in a solution that is slightly hypertonic. To avoid this, the invention provides a method for maintaining isotonicity by reconstituting a lyophilized formulation with a sodium chloride solution in a volume that is greater than the volume of the formulation pre-lyophilization.

The lyophilized factor IX formulation and sodium chloride solution of the invention are for use in a method for preparing a lyophilized Factor IX formulation for intravenous injection, the method comprising adding a 25 mM to 150 mM sodium chloride solution to the lyophilized Factor IX formulation thereby resulting in a formulation prepared for intravenous injection, wherein the prepared formulation is about isotonic with respect to plasma or is slightly hypotonic or slightly hypertonic with respect to plasma, and wherein the prepared formulation has a sufficient ionic strength to prevent erythrocyte agglutination. In one aspect, the lyophilized Factor IX formulation, when measured as if reconstituted in water, comprises (a) from about 5 mM to about 30 mM histidine; (b) from about 0.1M to about 0.3M glycine; (c) from about 0.5 to about 2 percent sucrose; (d) from about 0.001 to about 0.05 percent polysorbate; and (e) from about 0.4 mg/mL to about 20 mg/mL of Factor IX, or from about 0.1 mg/mL to about 100 mg/mL or some other soluble amount of Factor IX, or from about 10 IU/mL to about 500 IU/mL of Factor IX, or from about 10 IU/mL to about 5000 IU/mL of Factor IX. In one aspect, an about 40 mM sodium chloride solution is added to the lyophilized Factor IX formulation. In one aspect, about 5 mL of the about 40 mM sodium chloride solution is added to the lyophilized Factor IX formulation. In one aspect, the lyophilized Factor IX formulation if measured as if it is reconstituted in water comprises about 10 mM histidine, about 0.26M glycine, about 1% sucrose, and about 0.005% polysorbate.

In one aspect, the invention provides a pharmaceutical kit comprising: (a) a vial containing a lyophilized cake, wherein if the lyophilized cake is reconstituted in about 5 mL of water the solution would comprise: (i) from about 5 mM to about 30 mM histidine; (ii) from about 0.1M to about 0.3M glycine; (iii) from about 0.5 to about 2 percent sucrose; (iv) from about 0.001 to about 0.05 percent polysorbate; and (v) from about 0.4 mg/mL to about 20 mg/mL of Factor IX, or from about 0.1 mg/mL to about 100 mg/mL or some other soluble amount of Factor IX, or from about 50 IU/mL to about 500 IU/mL of Factor IX, or from about 10 IU/mL to about 5000 IU/mL of Factor IX; (b) a 25 mM to 150 mM sodium chloride solution; and (c) instructions for reconstituting the lyophilized cake with the sodium chloride solution, such that after reconstitution the resultant solution is about isotonic and has a sufficient ionic strength to prevent erythrocyte aggregation upon intravenous injection.

In one aspect, the invention provides a pharmaceutical kit comprising: (a) a vial containing a lyophilized cake, wherein if the lyophilized cake is reconstituted in 4 mL of water the solution would comprise: (i) about 10 mM histidine; (ii) about 0.26M glycine; (iii) about 1 percent sucrose; (iv) about 0.005 percent polysorbate 80; and (v) from about 50 IU/mL to about 5000 IU/mL of Factor IX; (b) an about 40 mM sodium chloride solution; and (c) instructions for reconstituting the lyophilized cake in the vial with about 5 mL of an about 40 mM sodium chloride solution, such that after reconstitution the resultant solution comprises: (i) from about 7 or 8 to about 10 mM histidine; (ii) from about 200 to about 210 mM glycine; (iii) from about 0.7% to about 0.9% sucrose; (iv) about 0.004% polysorbate 80; (v) from about 50 IU/mL to about 5000 IU/mL of Factor IX; and (vi) about 40 mM NaCl.

### BRIEF DESCRIPTION OF THE DRAWINGS

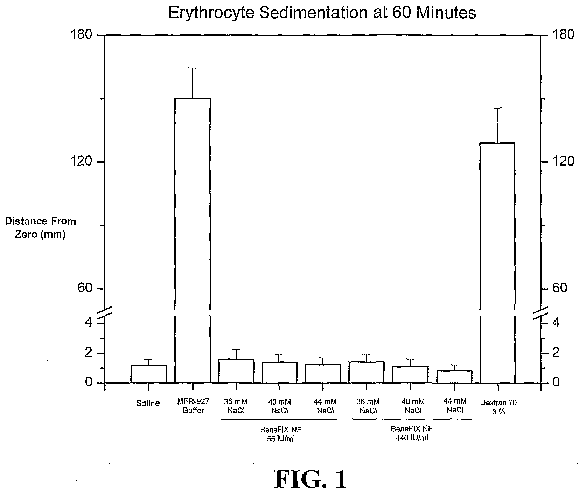

**Figure 1** shows erythrocyte sedimentation results from experiments described in Example 3. Erythrocyte sedimentation was measured at 60 minutes using an adaptation of the modified Westergren method (see Example 2), in which human blood collected in EDTA was mixed 1:4 with test solutions. After 60 minutes, the distance in mm between the zero mark and the erythrocyte:plasma interface was measured. Horizontal bars represent the mean and vertical brackets the standard deviation from a total of 12 donors. Results were combined from 4 independent experiments each of which evaluated blood from 3 donors.

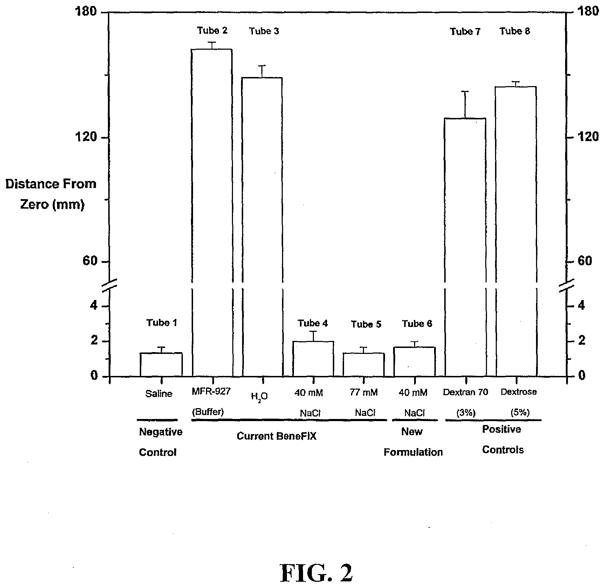

**Figure 2** shows an erythrocyte sedimentation evaluation on BeneFIX® formulations reconstituted with NaCl solutions. A 40 mM NaCl solution is sufficient to prevent erythrocyte agglutination when used to reconstitute either the currently marketed BeneFIX® product or a new formulation of BeneFIX® (BeneFIX®-R where prior to lyophilization the fill solution was comprised of 4 mL and had concentrations of 10 mM histidine, 260 mM glycine, 1% sucrose, 0.005% polysorbate 80; and after lyophilization it was reconstituted in 5 mL of 40 mM sodium chloride such that after reconstitution BeneFIX®-R comprises 40 mM NaCl, 8 mM histidine, 208 mM glycine, 0.8% sucrose, and 0.004% polysorbate).

### DETAILED DESCRIPTION OF THE INVENTION

The invention provides a lyophilized factor IX formulation and a sodium chloride solution as defined in claim 1 for use in methods for preparing pharmaceutical formulations for injection (in particular, preparations readied for intravenous injection) that do not cause erythrocyte agglutination, hemolysis, and/or cell shrinkage. To prevent agglutination, a pharmaceutical formulation ready for injection needs to have sufficient ionic strength. To prevent hemolysis or cell shrinkage, a pharmaceutical formulation ready for injection needs to be about isotonic with respect to plasma. The invention provides a lyophilized factor IX formulation and a sodium chloride solution as defined in claim 1 for use in methods that prepare pharmaceutical formulations for injection that have both the sufficient ionic strength to prevent agglutination and the requisite tonicity to prevent significant hemolysis or cell dehydration or shrinkage. The present methods involve the use of sodium chloride solutions that are 25 mM to 150 mM for reconstituting lyophilized cakes into solution. The addition of particular sodium chloride solutions result in a pharmaceutical preparation for injection that is about isotonic with respect to plasma or blood and is of sufficient ionic strength to prevent erythrocyte aggregation upon injection, in particular, upon intravenous injection.

### Terms

As used herein, the "molality" of a solution is the number of moles of a solute per kilogram of solvent.

As used herein, the "molarity" of a solution is the number of moles of solute per liter of solution.

As used herein, an "osmole" is the amount of a substance that yields, in ideal solution, that number of particles (Avogadro's number) that would depress the freezing point of the solvent by 1.86K.

As used herein, the "osmolality" of a solution is the number of osmoles of solute per kilogram of solvent Osmolality is a measure of the number of particles present in solution and is independent of the size or weight of the particles. It can be measured only by use of a property of the solution that is dependent only on the particle concentration. These properties are vapour pressure depression, freezing point depression, boiling point elevation, and osmotic pressure, and are collectively referred to as colligative properties.

As used herein, the "osmolarity" of a solution is the number of osmoles of solute per liter of solution.

### Ionic Strength

Ionic strength is a characteristic of an electrolyte solution (a liquid with positive and negatively charged ions dissolved in it). It is typically expressed as the average electrostatic interactions among an electrolyte's ions. An electrolyte's ionic strength is half of the total obtained by multiplying the molality (the amount of substance per unit mass of solvent) of each ion by its valence squared.

Ionic strength is closely related to the concentration of electrolytes and indicates how effectively the charge on a particular ion is shielded or stabilized by other ions (the so-called ionic atmosphere) in an electrolyte. The main difference between ionic strength and electrolyte concentration is that the former is higher if some of the ions are more highly charged. For instance, a solution of fully dissociated (broken down) magnesium sulfate (Mg²⁺SO₄²⁻) has 4 times higher ionic strength than a solution of sodium chloride (Na⁺Cl⁻) of the same concentration. Another difference between the two is that ionic strength reflects the concentration of free ions, and not just of how much salt was added to a solution. Sometimes a salt may be dissolved but the respective ions are still bound together pairwise, resembling uncharged molecules in solution. In this case the ionic strength is much lower than the salt concentration.

For the invention, pharmaceutical preparations are prepared for injection such that they are not only isotonic, but also have a sufficient ionic strength to prevent RBC agglutination. A sufficient ionic strength of a preparation ready for injection (*i.e.,* a lyophilized cake that is reconstituted with a NaCl solution of the invention) has at least 25 milliequivalents per liter (mEq/L) of Na⁺ and Cl⁻ ions. In one embodiment, a sufficient ionic strength is at least about 30, 31, 32, 33, 34, 35, 36, 37, 38, 39, or at least about 40 mEq/L of Na⁺ and Cl⁻ ions.

Ionic strength can also be described in terms of the conductivity of a solution. Conductivity is the ability of a material or solution to conduct electric current. The principle by which instruments measure conductivity is simple - two plates are placed in the sample, a potential is applied across the plates (normally a sine wave voltage), and the current is measured. Conductivity (G), the inverse of resistivity (R) is determined from the voltage and current values according to Ohm's law. G = I/R = I (amps) / E (volts)

Since the charge on ions in solution facilities the conductance of electrical current, the conductivity of a solution is proportional to its ion concentration. In some situations, however, conductivity may not correlate directly to concentration. However, for sodium chloride solutions, conductivity is directly proportional to ion concentration. The basic unit of conductivity is the siemens (S), formerly called the mho. Since geometry of a test sample affects conductivity values, standardized measurements are expressed in specific conductivity units (S/cm) to compensate for variations in electrode dimensions. Specific conductivity (C) is simply the product of measured conductivity (G) and the electrode cell constant (L/A), where L is the length of the column of liquid between the electrode and A is the area of the electrodes. C = G x (L/A). If the cell constant is 1 cm⁻¹, the specific conductivity is the same as the measured conductivity of the solution. Although electrode shape varies, an electrode can always be represented by an equivalent theoretical cell.

Thus, in one embodiment, a sufficient ionic strength of a preparation ready for injection can have, for example, at least about a conductivity of about 4 mS/cm or higher. In another embodiment, the sufficient ionic strength of a solution ready for injection is at least about 2.5, 2.6, 2.7, 2.8, 2.9, 3.0, 3.1, 3.2, 3.3, 3.4, 3.5, 3.6, 3.7, 3.8, or at least about 3.9 mS/cm.

An operational definition of whether a solution has a sufficient ionic strength to prevent erythrocyte agglutination can also be used to explain the term "sufficient ionic strength." For example, this can be based on an experiment of adding a test solution to whole blood and observing the distance of erythrocyte sedimentation (see Example 2; adapted, modified Westergren method). In one embodiment, if a test solution when mixed with whole blood in a ratio of 4:1 provides an erythrocyte sedimentation at 60 minutes that is less than about 10 mm (see Example 2, Figures 1 and 2, for example), then the test solution has a sufficient ionic strength to prevent erythrocyte agglutination. In another embodiment, if a test solution when mixed with whole blood in a ratio of 4:1 provides an erythrocyte sedimentation at 60 minutes that is less than about 5 mm, then the test solution has a sufficient ionic strength to prevent erythrocyte agglutination.

### Solution Osmolarity

The methods of the invention provide pharmaceutical preparations for injection that are about isotonic with respect to blood. To determine whether a pharmaceutical preparation is about isotonic with respect to blood, one calculates the osmolarity for all chemical components of a solution including the diluent. The osmolar concentration of pharmaceutical preparations for injection (parenteral solutions) can exert adverse effects on the blood cells and vessels of the human body. Tonicity can be calculated for fluids and dissolved or diluted medications, which are expressed in a numerical value of milliosmoles per liter of fluid (mOsm/L). This value is also known as osmolarity. The osmolarity of blood ranges between 285 and 310 mOsm/L. When hypotonic or hypertonic solutions are injected into blood, fluid shifts into or out of cells, which can cause a variety of negative effects.

Solution osmolarity is based in part on the concepts of osmosis and osmotic pressure. Osmosis is the diffusion of solutes (dissolved particles) or the transfer of fluid through semipermeable membranes such as blood vessels or cell membranes. Osmotic pressure, which facilitates the transport of molecules across membranes, is expressed in osmolar concentrations and is referred to as hypo-osmotic (hypotonic), iso-osmotic (isotonic), or hyper-osmotic (hypertonic) when compared with biologic fluids such as blood or plasma. The term "tonicity" and "osmotic pressure" are often considered to be synonymous.

The osmotic pressure is the hydrostatic (or hydraulic) pressure required to oppose the movement of water through a semipermeable membrane in response to an 'osmotic gradient' (*i.e.,* differing particle concentrations on the two sides of the membrane). Serum osmolality can be measured by use of an osmometer or it can be calculated as the sum of the concentrations of the solutes present in the solution. The value measured in the laboratory is usually referred to as the osmolality. The value calculated from the solute concentrations is reported by the laboratory as the osmolarity. The osmolar gap is the difference between these two values.

. Herein, tonicity and osmotic pressure are to be considered synonymously, and are to be understood broadly. Tonicity can mean the effective osmolality and is equal to the sum of the concentrations of the solutes in a solution that have the capacity to exert an osmotic force across a membrane, including a cell membrane. In the strict sense, osmolality is a property of a particular solution and is independent of any membrane. Tonicity is a property of a solution in reference to a particular membrane. However, the invention shall refer to solutions being isotonic with respect to biological solutions such as blood or plasma, and this referencing shall include the meaning that the particular solution is isotonic with blood or plasma with respect to a cell membrane of a cell in the blood or plasma or other biological solution.

An operational definition of tonicity can be used to explain the term. This can be based on an experiment of adding a test solution to whole blood and observing the result. If the RBCs in whole blood swell and rupture, the test solution is said to be hypotonic compared to normal plasma. If the RBCs shrink and become crenated, the test solution is said to be hypertonic compared to normal plasma. If the RBCs stay the same, the test solution is said to be isotonic with plasma. The RBC cell membrane is the reference membrane. For example, whole blood placed in normal saline (*i.e.,* 0.9% sodium chloride) will not swell, and hence normal saline is said to be isotonic.

### Characteristics of Isotonic Solutions

The invention provides methods for preparing or readying pharmaceutical formulations for injection into a subject, wherein the formulations are prepared into solutions that are (1) about isotonic with respect to blood (or plasma or other biologic fluid) or are not so hypertonic or hypotonic as to cause significant hemolysis, thrombosis, or vessel irritation, and (2) have sufficient ionic strength to prevent erythrocyte aggregation.

In one embodiment, isotonic (with respect to blood) pharmaceutical formulations ready for injection have a tonicity or osmolarity that is greater than 270 mOsm/L and less than 330 mOsm/L. In one embodiment, the isotonic pharmaceutical formulations ready for injection have a tonicity or osmolarity that is greater than 270 mOsm/L and less than 328 mOsm/L.

Although solutions such as 0.9% sodium chloride and 5% dextrose are isotonic, when they are used to reconstitute or dilute many pharmaceutical formulations, the resultant solution may not have sufficient ionic strength to prevent erythrocyte agglutination (as for 5% dextrose) or may have too much ionic strength such that the resultant solution is hypertonic. Thus, the methods of the invention use solutions for dilution or reconstitution that contain a minimum concentration of sodium chloride to provide (a) a sufficient ionic strength to mitigate erythrocyte aggregation, and (b) a sufficient tonicity to prevent hemolysis, and a maximum concentration of sodium chloride to provide (c) a resultant tonicity that is not so great as to be hypertonic solutions. Further, the methods of the invention use solutions for dilution or reconstitution that can provide a sufficient ionic strength and isotonicity with respect to blood, while also maintaining a practical injection volume for the pharmaceutical preparation.

### Characteristics of Hypotonic Solutions

The invention provides methods for preparing pharmaceutical formulations for injection into a subject, wherein the formulations are prepared into solutions that are not hypotonic with respect to blood or are not so hypotonic (*i.e.,* slightly hypotonic with respect to blood) so as to cause significant hemolysis. In one embodiment, the pharmaceutical formulations ready for injection that is considered slightly hypotonic with respect to blood can have a tonicity or osmolarity that is less than 270 mOsm/L and greater than 240 mOsm/L. In one embodiment, the pharmaceutical formulations ready for injection that is considered slightly hypotonic with respect to blood can have a tonicity or osmolarity that is less than 270 mOsm/L and greater than 220 mOsm/L.

Examples of hypotonic solutions include many pharmaceutical preparations that are readied for injection with sterile water. When hypotonic solutions are injected, a fluid shift occurs and water is moved into the endothelial cells of the vein and blood cells. Cells that absorb too much water can burst, and thus, injection of hypotonic solutions can cause vein irritation, phlebitis, and hemolysis.

### Characteristics of Hypertonic Solutions

The invention provides methods for preparing pharmaceutical preparations for injection into a subject, wherein the preparations are prepared into solutions that are not hypertonic with respect to blood or are not so hypertonic (*i.e.,* slightly hypertonic) as to cause significant thrombosis and/or vessel irritation. In one embodiment, the pharmaceutical formulations ready for injection that is considered to be slightly hypertonic can have a tonicity or osmolarity that is greater than 340 mOsm/L and less than 600 mOsm/L. In one embodiment, the pharmaceutical formulations ready for injection that is considered to be slightly hypertonic can have a tonicity or osmolarity that is greater than 340 and less than 375, 400, 425, 450, 475, 500, or 575 mOsm/L. In general, hypertonic solutions exhibit a tonicity that is greater than 340 mOsm/L. Solutions with an osmolarity that is greater than 600 mOsm/L should be used with care in injections.

Examples of undesirable hypertonic solutions include many pharmaceutical formulations that are readied for injection with 10% dextrose, or pharmaceutical preparations that have multiple additives that affect osmolarity. When hypertonic solutions are injected, a fluid shift occurs and water is drawn out of the endothelial cells of the vein and blood cells. Cells that lose too much water can shrink, and thus, injection of hypertonic solutions can cause vein irritation, phlebitis, and thrombosis.

### pH

The pH of blood ranges from about 7.35 to about 7.45, which is considered neutral. Pharmaceutical preparations with a pH value below 7 are considered acidic drugs and those with a pH value below 4.1 are considered very acidic. Drugs with a pH value higher than 7.5 are considered basic or alkaline drugs, and those with a pH value higher than 9.0 are considered very basic or alkaline. Very acidic or very alkaline drug solutions can cause phlebitis and thrombosis. Thus, in one embodiment, the invention provides sodium chloride solutions for reconstituting lyophilized drug formulations or for diluting liquid drug formulations to ready these formulations for intravenous injection, wherein the readied preparations for injection (1) do not have a pH that is less than 4.1 or greater than 9.0, (2) have a sufficient ionic strength to prevent erythrocyte aggregation, and (3) is about isotonic (or slightly hypertonic or hypotonic) with respect to blood such that hemolysis or crenation does not occur to RBCs. However, the pH of a solution is not a consideration with respect to whether the solution has a sufficient ionic strength to prevent erythrocyte aggregation. In other words, if a lyophilized formulation when reconstituted with water has a pH that is below 4.1 or greater than 9.0, this does not prevent the use of a sodium chloride solution for reconstitution in order to prevent erythrocyte agglutination.

### Calculating Osmolarity

The osmolarity of any pharmaceutical preparation can be calculated by using the following formula: Osmolarity = (weight of substance (g) divided by the molecular weight of the substance (g/L)) multiplied by the number of species multiplied by 1000 for milliosmolarity. The term "species" refers to the number of ions or chemical species formed when dissolution occurs.

### Pharmaceutical Formulations Ready or Prepared for Injection

The invention provides methods for reconstituting lyophilized drug products into solution in order to prepare the drug product for injection into a subject. The reconstituted drug product is ready for injection by having a sufficient ionic strength and a tonicity that is about isotonic with respect to blood.

The methods of the invention also pertain to diluting drug solutions in order to prepare the drug solution for injection into a subject. The diluted drug solution is ready for injection by having a sufficient ionic strength and by being about isotonic with respect to blood.

In one embodiment, the lyophilized or dry drug product/formulation, if reconstituted in water, has an osmolarity of 100 mOsm/L to 360 mOsm/L. Because the invention provides methods for reconstitution using 25mM to 150 mM sodium chloride solutions, the practitioner should use a particular sodium chloride solution based upon the expected combined osmolarity of the lyophilized drug product reconstituted into the sodium chloride solution. Thus, for example, if a lyophilized drug product if reconstituted in water has an osmolarity of about 300 mOsm/L, then the NaCl solution for reconstitution should be 25 mM to 30 mM. In another example, if a lyophilized drug product if reconstituted in water has an osmolarity of about 100 mOsm/L, then the NaCl solution for reconstitution should be 25 mM to 130 mM.

In one embodiment, a lyophilized drug product to be prepared for injection by the present methods does not contain HES (hydroxyethyl starch). HES-containing formulations, despite reconstitution or dilution with NaCl solutions for ionic strength, can cause erythrocyte sedimentation and agglutination in *in vitro* experiments. In another embodiment, a drug product to be prepared for injection by the present methods does not contain dextrans, because adapted modified Westergren methods (see Example 2) show that the addition of NaCl would not counteract the effect of enhanced sedimentation that dextrans can cause.

In one embodiment, the invention provides a lyophilized factor IX formulation and a sodium chloride solution for use in a method for preparing a pharmaceutical preparation for intravenous injection wherein the pharmaceutical preparation is a lyophilized cake comprising a primary bulking agent. The primary bulking agent can be, for example, mostly non-ionizing. Non-ionizing bulking agents include, but are not limited to, mannitol, glycine, sucrose, lactose, other disaccharides, therapeutic proteins or the active ingredient of a formulation itself, or other bulking agents known to one skilled in the art. The concentrations of non-ionizing bulking agents do not significantly affect whether a solution has a sufficient ionic strength to prevent agglutination. However, their concentrations do have an effect on osmolarity, and therefore, their concentrations can have an effect on tonicity. The concentration of glycine, for example, is equivalent to its contribution to osmolarity, thus 10 mM glycine is equivalent to 10 mOsm/L.

Protein ingredients in a drug formulation, including active ingredients, do not significantly affect ionic strength of a solution or the osmolarity of a solution. For example, assume a molecular weight of 50,000 for a protein and assume 2.5 mg of the protein in a 1-2 mL solution to be injected. This is equivalent to 0.05 mM, thus, the protein does not appreciably contribute to osmolarity.

Pharmaceutical preparations also often contain surfactants, such as polysorbate-80. Polysorbate-80 and other surfactants are large molecular weight molecules, so amounts that are usually present in preparations, such as 0.001% to 0.01%, are too small to contribute appreciably to osmolarity or ionic strength. Other surfactants include Brij® 35, Brij® 30, Lubrol-px™, Triton X-10, Pluronic® F127, and sodium dodecyl sulfate (SDS).

Other large molecular weight molecules that may be present in small amounts in pharmaceutical formulations that do not appreciably affect osmolarity or ionicity are polymers, with the qualification that they are not salts like dextran sulfate. Exemplary polymers include dextran, poly(vinyl alcohol) (PVA), hydroxypropyl methylcellulose (HPMC), gelatin, polyethylene glycol (PEG) and polyvinylpyrrolidone (PVP).

Pharmaceutical formulations also often contain sucrose or other sugars or polyols, often in an amount of 0-2, 0-5, or 0-10% or higher. For example, 5-10% sucrose can be used when the formulation does not comprise a bulking agent. Generally, where a formulation is lyophilized, and amounts of the formulation are identified herein as percentages or molarity amounts, this is in reference to percentages and molarity of a solution prior to lyophilization (*i.e.,* fill amounts). Thus, when a solution has 1% sucrose, this is equivalent to 29.2 mM. Because sucrose does not appreciably ionize or disassociate in solution, 29 mM of sucrose is equivalent to 29 mOsm/L. Other sugars or polyols that do not appreciably ionize or disassociate in solution include glycerol, xylitol, sorbitol, mannitol (also can be used as a bulking agent), glucose, inositol, raffinose, maltotriose, lactose, and trehalose.

Pharmaceutical formulations also can contain buffering agents. Buffering agents include, for example, acetate, citrate, glycine, histidine, phosphate (sodium or potassium), diethanolamine and Tris. Buffering agents include those agents that maintain a solution pH in an acceptable range. Buffering agents such as glycine, histidine, and diethanolamine are mostly non-ionizing, and thus their concentrations are equivalent to osmolarity and should be kept in mind when considering whether a formulation ready for injection is about isotonic. Buffering agents such as acetate and citrate are usually salts, and are therefore ionizing, and their concentrations are multiplied with respect to calculating their contribution to a solution's osmolarity.

Pharmaceutical formulations can include essentially any active ingredient, including proteins, nucleic acids, viruses, and chemical compounds. These molecules mostly do not appreciably affect the ionic strength or the osmolarity of a solution to be injected. If these molecules are salts, as often small molecule compounds are pharmaceutical salts, then they may appreciably affect the ionic strength and/or osmolarity.

Certain amino acids are also found in some pharmaceutical formulations and are used as cryoprotectants, lyoprotectants and/or bulking agents. Some amino acids, such as histidine, are mostly non-ionizing, and thus the concentration of an amino acid in a formulation should only be kept in mind when calculating the osmolarity of a solution such that a formulation ready for injection is about isotonic with respect to blood.

### BeneFIX®

BeneFIX® is produced by a genetically engineered Chinese hamster ovary (CHO) cell line that is extensively characterized and shown to be free of infectious agents. The stored cell banks are free of blood or plasma products. The CHO cell line secretes recombinant Factor IX (rFIX) into a defined cell culture medium that does not contain any proteins derived from animal or human sources, and the recombinant Factor IX is purified by a chromatography purification process that does not require a monoclonal antibody step and yields a high-purity, active product. A membrane filtration step that has the ability to retain molecules with apparent molecular weights >70,000 (such as large proteins and viral particles) is included for additional viral safety. BeneFIX® is predominantly a single component by SDS-polyacrylamide gel electrophoresis evaluation. The potency (in international units, I.U. (IU)) is determined using an *in vitro* one-stage clotting assay against the World Health Organization (WHO) International Standard for Factor IX concentrate. One international unit is the amount of Factor IX activity present in 1 mL of pooled, normal human plasma. The specific activity of BeneFIX® is greater than or equal to 180 IU per milligram of protein. BeneFIX® is not derived from human blood and contains no preservatives or added animal or human components.

BeneFIX® is formulated as a sterile, nonpyrogenic, lyophilized powder preparation. BeneFIX® is intended for intravenous (IV) injection. It is available in single use vials containing the labeled amount of Factor IX activity, expressed in international units (IU). Each vial contains, for example, nominally 250, 500, or 1000 IU (or more, including 2000 IU) of coagulation Factor IX (Recombinant). After reconstitution of the lyophilized drug product with sterile water, the concentrations of excipients in the 500 and 1000 IU dosage strengths are 10 mM L-histidine, 1% sucrose, 260 mM glycine, 0.005% polysorbate 80. The concentrations after reconstitution in the 250 IU dosage strength are half those of the other two dosage strengths. The 500 and 1000 IU dosage strengths are isotonic after reconstitution, and the 250 IU dosage strength has half the tonicity of the other two dosage strengths after reconstitution. All dosage strengths yield a clear, colorless solution upon reconstitution.

In one embodiment, the invention provides a lyophilized factor IX formulation and a sodium chloride solution for use in a method for preparing BeneFIX® to be injected into a subject, where BeneFIX® is reconstituted into a sodium chloride solution, wherein the sodium chloride solution is greater than 25 mM and less than 150 mM. In one embodiment, the sodium chloride solution used to reconstitute BeneFIX® is about 40 mM. In one embodiment, one vial of lyophilized BeneFIX® is reconstituted into about 4-5 mL of a 25mM-150mM sodium chloride solution.

### Reformulated BeneFIX® (BeneFIX®-R)

Because of the low ionic strength of BeneFIX® reconstituted in water, various reformulations were made (Reformulated BeneFIX® (BeneFIX®-R)). The goal was to add sufficient ionic strength to the BeneFIX® formulation so as to mitigate the potential for RBC agglutination. In order to increase the ionic strength of BeneFIX®, sodium chloride can be incorporated into the reconstituted product by replacing the sWFI as the reconstituting solution. Alternatively, BeneFIX® can be reformulated by adding NaCl to the pre-lyophilization formulation, such that reconstitution can still be achieved with water, but lyophilizing salt solutions is more difficult, and it is more practical to simply reconstitute lyophilized cakes with NaCl solutions. This is also true for other lyophilized pharmaceutical preparations that do not have a sufficient ionic strength when reconstituted with sWFI to prevent agglutination.

In one embodiment, BeneFIX®-R can be lyophilized in the same formulation as BeneFIX® (10 mM L-histidine, 260 mM glycine, 1% sucrose, 0.005% polysorbate 80), and only the rFIX concentration will differ. BeneFIX®-R bulk drug product can then be filled, for example, at 4 mL per vial with 10 mM L-histidine, 260 mM glycine, 1% sucrose, 0.005% polysorbate 80, and lyophilized. When BeneFIX®-R is reconstituted to 5 mL per vial with a 25-150 mM NaCl solution, such as with 40 mM NaCl, the concentration of the excipients is reduced by 20% when compared to the current BeneFIX® formulation pre-lyophilization. This strategy results in a formulation that is (1) isotonic, (2) has sufficient ionic strength to reduce the potential for RBC agglutination, and (3) reduces the injection volume for higher doses of rFIX. BeneFIX®-R can be provided with, for example, 250, 500, 1000, and 2000 IU of rFIX per vial dosage strengths. Thus, after reconstitution with 5 mL of a 25-150 mM NaCl solution, the resultant BeneFIX®-R formulation solution can be from about 7 to about 9 mM histidine; from about 188 to about 220 mM glycine; from about 0.7% to about 0.9% sucrose; and about 0.004% polysorbate 80. Depending upon the amount of rFIX in a vial, for example, 250, 500, 1000, or 2000 IU, reconstitution in 5 mL of a NaCl solution would result in an rFIX concentration of about 50, 100, 200, or 400 IU/mL, respectively. The osmolarity of the BeneFIX®-R formulation reconstituted in 5 mL of a 40 mM NaCl solution is about 320 mOsm/L.

### Exemplary Formulations To Be Prepared for Injection

Recombinant Factor IX can also be lyophilized in formulations described in U.S. Patent No. 6,372,716. The formulations described in the patent can be used with the present invention. Thus, for example, lyophilized formulations that can be reconstituted with 25 mM-150 mM sodium chloride solutions to prepare them for injection, include formulations that comprise glycine, polysorbate, sucrose, histidine, and an active ingredient. The glycine can have a concentration, for example, of about 0.1M to 0.3M. The polysorbate can have a concentration, for example, of about 0.001 to about 0.05%. The sucrose can have a concentration, for example, of about 0.5% to about 2%. The histidine can have a concentration, for example, of about 5 mM to about 30 mM.

In one embodiment, a lyophilized formulation to be reconstituted with 25-150 mM sodium chloride solution comprises about 0.1 to 0.3M glycine, about 0.5 to 2% sucrose, 0.001 to about 0.05% polysorbate, about 5 to about 30 mM histidine, and about 0.1 to about 20 mg/mL of an active ingredient. The active ingredient can be, for example, rFIX. In another embodiment, a rFIX lyophilized formulation to be reconstituted with 25-150 mM sodium chloride solution comprises about 0.13 to about 3 mg/ml rFIX or about 50 to about 600 IU/mL of rFIX, about 0.26M glycine, about 10 mM histidine, about 1% sucrose, and about 0.005% polysorbate.

### EXAMPLES OF THE INVENTION

The examples described below are provided to illustrate aspects of the present invention and are not included for the purpose of limiting the invention.

### Example 1: Effects of Anti-Coagulants and Formulation Components on BeneFIX®-Associated RBC Agglutination

There are occasional reports of BeneFIX®-associated RBC agglutination in butterfly catheter lines and syringes. Recent studies in blood from hemophilia dogs demonstrate that the agglutination occurs in the absence of recombinant human FIX in the formulation buffer. Thus, the present study investigated whether standard anti-coagulants, various BeneFIX® components, and ionic strength affects the BeneFIX®-associated agglutination phenomenon. In this example, only combinations of lyophilized factor IX formulations with 25 mM to 150 mM sodium chloride solution are according to the present claims. The other tested samples are for reference.

Blood was obtained from a pool of anonymous human volunteers and was collected into Vacutainer tubes (Becton Dickinson, Franklin Lakes, NJ) containing a standard anti-coagulant, such as ethylenediamine tetra-acetic acid (EDTA), sodium citrate, or heparin.

To test for agglutination, the blood samples in the Vacutainer tubes were first continuously mixed on a nutator. Blood, 12.5 µL, was diluted in 87.5 µL of a test solution in a 48-well cell culture plate, and was allowed to incubate at room temperature for 2 minutes prior to observation under an inverted-phase contrast microscope at 400x magnification. Each well was videotaped to capture still images in order to score RBC agglutination according to the following criteria listed below in Table 1.

**Table 1: RBC Agglutination Scoring Key**

| **Score** | **Agglutination Phenotype** |
|---|---|
| 0 | No agglutination, majority are individual cells, few or occasional doublets. |
| 1 | Mostly individual cells, small aggregates dispersed throughout. |
| 2 | Lots of small aggregates, still some individual cells in background. |
| 3 | Mostly small to medium aggregates with occasional individual cells. |
| 4 | Massive cell aggregates. |

*Effects of Standard Anti-Coagulants on RBC Agglutination:* The first study included the assay of blood samples from three donors. Blood was collected into EDTA, heparin, and sodium citrate Vacutainer tubes for each donor. The test articles included BeneFIX®, 100 IU/mL, reconstituted in each of the following NaCl concentrations: 0 mM (sWFI), 20 mM, 40 mM, 60 mM, and 77 mM NaCl. In addition, dextrose 5% in water (D5W) was included as a positive control. The samples were diluted at a blood to BeneFIX® or blood to D5W ratio of 1:8. Images were captured, stored digitally, masked, and scored for agglutination. Two minutes following the addition of blood to the BeneFIX® reconstituted with sWFI, agglutination was seen in the samples from all three donors regardless of the type of anti-coagulant used. As increasing concentrations of NaCl were used, the agglutination response was attenuated. Marked agglutination was seen when blood was added to D5W.

*Effects of NaCl on RBC Agglutination in Pre-screened Donors:* Donor samples were collected into heparinized Vacutainer tubes. Because spontaneous agglutination had been observed in some previous samples, all donors were initially screened using 154 mM NaCl. If agglutination was observed, the sample was disqualified from further assays. The remaining samples from each donor were diluted 1:8 into one of two formulations: (1) 100 IU/mL BeneFIX® reconstituted with sWFI, and (2) 100 IU/mL BeneFIX® reconstituted with 154 mM NaCl. Images were captured, stored digitally, masked and scored for agglutination. Agglutination was seen with BeneFIX® reconstituted sWFI, whereas reconstitution with 154 mM NaCl reduced or eliminated the agglutination reaction (see Table 2 for scores).

**Table 2: Agglutination Scores**

| **Donor** | **154 mM NaCl Pre-Screen Control** | **BeneFIX® + sWFI** | **BeneFIX® + 154 mM NaCl** |
|---|---|---|---|
| 7 | 0 | 2 | 1 |
| 11 | 0 | 3 | 0 |
| 8 | 0 | 3 | 0 |
| 28 | 0 | 3 | 0 |
| 41 | 0 | 3 | 1 |

*Effect of Formulation Components and BeneFIX*® *Concentration:* Several formulations of BeneFIX® were evaluated to ascertain the impact of different components on RBC agglutination. Eight different mixtures, with and without 154 mM NaCl were prepared, for a total of 16 mixtures (Table 3). Twelve donors were screened for spontaneous agglutination and two were disqualified from further study. Blood samples from five of the ten remaining donors were used for the first 8 formulations (samples 1-A through 1-H) and the other five donor samples were used for the second 8 formulations (samples 2-1 through 2-P). Samples were diluted 1:8 as before and images were captured and scored as previously described.

**Table 3: Formulation Design Matrix**

| (+/indicates presence/absence of an ingredient) | | | | | | |
|---|---|---|---|---|---|---|
| **Set/Sample ID** | **BeneFIX® concentration (IU/mL)** | **Glycine (GLY) 260 mM** | **Sucrose 10mg/mL** | **Tween-80 (Polysorbate-80) 0.005%** | **Histidine (HIS) 10 mM** | **NaCl 154mM** |
| 1-A, BeneFIX® | 100 | + | + | + | + | - |
| 1-B, BeneFIX® | 100 | + | + | + | + | + |
| 1-C, BeneFIX® | 300 | + | + | + | + | - |
| 1-D, BeneFIX® | 300 | + | + | + | + | + |
| 1-E, BeneFIX® | 600 | + | + | + | + | - |
| 1-F, BeneFIX® | 600 | + | + | + | + | + |

| **Set/Sample ID** | **BeneFIX® concentration (IU/mL)** | **Glycine (GLY) 260 mM** | **Sucrose 10mg/mL** | **Tween-80 (Polysorbate-80) 0.005%** | **Histidine (HIS) 10 mM** | **NaCl 154mM** |
|---|---|---|---|---|---|---|
| 1-G, No BeneFIX® | - | + | + | + | + | - |
| 1-H, No BeneFIX® | - | + | + | + | + | + |
| 2-1, BeneFIX®, No GLY | 100 | - | + | + | + | - |
| 2-J, BeneFIX®, No GLY | 100 | - | + | + | + | + |
| 2-K, No Sucrose | 100 | + | - | + | + | - |
| 2-L, No Sucrose | 100 | + | - | + | + | + |
| 2-M, BeneFIX®, No Tween-80 | 100 | + | + | - | + | - |
| 2-N, No Tween-80 | 100 | + | + | - | + | + |
| 2-O, BeneFIX® No HIS | 100 | + | + | + | - | - |
| 2-P, BeneFIX® No HIS | 100 | + | + | + | - | + |

Agglutination scores for the experiment laid out in Table 3 are shown below in Tables 4A and 4B. No particular component, whether the active ingredient or an excipient, of the BeneFIX® preparation was associated with agglutination. As expected, removal of glycine caused RBC lysis due to hypotonicity of the solution. However, reconstitution with 154 mM NaCl reduced or eliminated the in vitro agglutination and the lysis.

**Table 4A: Agglutination Scores - Varying the BeneFIX® Concentration**

| **Sample Donor ID** | **1-A** | **1-B** | **1-C** | **1-D** | **1-E** | **1-F** | **1-G** | **1-H** |
|---|---|---|---|---|---|---|---|---|
| 6 | 4 | 1 | 2 | 1 | 4 | 1 | 3 | 2 |
| 14 | 3 | 40 | 2 | 3 | 3 | 1 | 2 | 4 |
| 19 | 4 | 1 | 3 | 40 | 3 | 1 | 3 | 1 |
| 40 | 1 | 40 | 2 | 1 | 2 | 2 | 3 | 1 |
| 43 | 3 | 40 | 3 | 1 | 4 | 40 | 3 | 3 |
| *Mean score* | 3 | 0.4 | 2.4 | 1.2 | 3.2 | 1 | 2.8 | 2.2 |

**Table 4B: Agglutination Scores - Excipients Effects**

| **Sample Donor ID** | **2-I** | **2-J** | **2-K** | **2-L** | **2-M** | **2-N** | **2-O** | **2-P** |
|---|---|---|---|---|---|---|---|---|
| 1 | Lysis | 1 | 2 | 40 | 3 | 1 | 3 | 40 |
| 11 | 4 | 40 | 4 | 1 | 3 | 1 | 2 | 40 |
| 18 | Lysis | 1 | 4 | 1 | 3 | 2 | 3 | 2 |
| 34 | Lysis | 0 | 2 | 0 | 2 | 2 | 3 | 2 |
| 45 | Lysis | 1 | 4 | 1 | 4 | 3 | 2 | 3 |
| *Mean score* | Lysis | 0.6 | 3.2 | 0.6 | 3 | 1.8 | 2.6 | 1.4 |

*Effects of Lower Concentrations of NaCl on RBC Agglutination in Pre-Screened Donors:* Donor samples were collected into heparinized Vacutainer tubes. Because agglutination had been observed in some previous samples, all donors were initially screened with 154 mM NaCl. If agglutination was observed, the sample was disqualified from further assays. The remaining samples from each donor were diluted 1:8 as before into one of three formulations: (1) 100 IU/mL BeneFIX® with sWFI; (2) 100 IU/mL BeneFIX® with 40 mM NaCl; or (3) 100 IU/mL BeneFIX® with 77 mM NaCl. Images were captured, collected and scored as before. The results are provided in Table 5 below. Agglutination was seen in all five samples with BeneFIX® reconstituted with water, whereas reconstitution of BeneFIX® with 40 mM or 77 mM NaCl reduced or eliminated the agglutination reaction.

**Table 5: Agglutination Scores**

| **Donor No.** | **154 mM NaCl** | **BeneFIX® + sWFI** | **BeneFIX® + 40 mM NaCl** | **BeneFIX® + 77 mM NaCl** |
|---|---|---|---|---|
| 8 | 0 | 3 | 1 | 2 |
| 34 | 0 | 3 | 2 | 1 |
| 39 | 0 | 4 | 3 | 1 |
| 40 | 0 | 3 | 0 | 1 |
| 47 | 0 | 3 | 1 | 1 |
| *Mean score* | 0 | 3.2 | 1.4 | 1.2 |

*Analysis:* RBC agglutination occurred in blood anti-coagulated with heparin, EDTA, or sodium citrate when mixed with BeneFIX® at a blood to reconstituted BeneFIX® volume ratio of 1:8. No effect of anti-coagulant type was seen on the agglutination; therefore heparin was used as the anti-coagulant in subsequent evaluations. In some samples, agglutination occurred in the presence of 0.9% NaCl alone (negative control for agglutination). Therefore, subsequent evaluations were conducted only with samples that exhibited no agglutination with the 0.9% NaCl control.

The effects of three concentrations of BeneFIX® (100, 300, or 600 IU/mL) and of each individual component of BeneFIX®, reconstituted in sWFI or NaCl solution were then evaluated for RBC agglutination when mixed with heparin anti-coagulated blood at a blood to BeneFIX® volume ratio of 1:8. Elimination of any particular component, including the recombinant human FIX protein had no effect on the agglutination response. This indicates that a broad range of different pharmaceutical formulations can be readied (reconstituted or diluted) with NaCl solutions for intravenous injection. Removal of glycine resulted in RBC lysis. However, the use of 40 mM (0.234% NaCl, injectable), 77 mM (0.45% NaCl, injectable) or 154 mM NaCl (0.9% NaCl, injectable) for reconstitution reduced or eliminated the agglutination response and lysis.

Thus, these results show that no particular component of the BeneFIX® formulation is related to the observed agglutination. Although removal of glycine from the BeneFIX® formulation and reconstitution with water results in a hypotonic solution that causes lysis, osmolarity and tonicity is a distinct concern from ionic strength and agglutination. The results show that agglutination is associated with the low ionic strength of the reconstituted recombinant Factor IX (rFIX) in sWFI, and reconstitution with NaCl attenuates or eliminates agglutination. Thus, reconstitution of other pharmaceutical formulations with NaCl solutions, even NaCl solutions having a concentration below 154 mM, can prevent both agglutination and lysis upon intravenous injection.

### Example 2: Adaptation of the Modified Westergren Method of Erythrocyte Sedimentation Rate Measurement to Assess Erythrocyte Aggregation Induced by Pharmaceutical Agents or Formulations

The currently marketed BeneFIX® formulation is a non-ionic formulation. During administration of the BeneFIX® formulation reconstituted in sWFI, there has been infrequent observations of erythrocyte aggregation (*i.e.,* agglutination) when a patient's blood is mixed with the reconstituted BeneFIX®, such as in intravenous tubing. The invention provides the discovery that erythrocyte aggregation is associated with the formulation, not rFIX, and is prevented by using diluents that contain at least about 40 mM NaCl. To assist in the design of custom diluents containing at least about 40 mM NaCl, a quantifiable assay was designed that can be used to measure erythrocyte sedimentation, which is an established method to assess erythrocyte aggregation in vitro. The modified Westergren method, in which blood is diluted 4:5 in normal saline, was adapted to assess aggregation in blood that had been diluted 1:4 or 1:8 with either saline or test solutions (*i.e*., custom diluents). Only combinations of lyophilized factor IX formulation and a 25 mM to 150 mM sodium chloride solution are according to the present claims. Other tested samples are for reference.

Human blood was obtained from healthy adult volunteers and collected into tubes containing EDTA. To demonstrate the suitability of blood samples for sedimentation experiments, the clinically defined erythrocyte sedimentation rate ESR₆₀ was measured by the modified Westergren method. Briefly, well-mixed blood was diluted 4:5 with 154 mM NaCl, gently mixed well, and then loaded immediately onto a self-zeroing Westergren tube that had been placed in a custom 10-tube rack. The distance in mm from the zero mark at the top of the tube to the plasma: erythrocyte interface after 60 minutes was measured and recorded. The ESR₆₀ results were compared with published normal reference values (Morris, M.W. et al., Basic examination of blood, in Henry, J.B., ed., Clinical Diagnosis and Management by Laboratory Methods, Philadelphia, PA, WB Saunders, 2001: 479-519).

Two experiments were conducted to show the suitability of the adapted, modified Westergren method to assess erythrocyte aggregation associated with pharmaceutical formulations. Human blood samples were suitable for use in these assays because all had ESR₆₀ values that were within normal limits. These samples were diluted 1:4 in the first experiment and diluted 1:8 in the second.

In the first experiment, erythrocyte sedimentation was enhanced by 5% dextran 70 or BeneFIX® reconstituted with sWFI or 10 mM NaCl. Within 5 minutes, aggregates were visible in the tubes that had been loaded with blood diluted with either 3% dextran 70 or BeneFIX® reconstituted in sWFI. By 60 minutes, erythrocyte sedimentation in these tubes was markedly enhanced when compared with saline control (Table 6). By 90 minutes, blood from one donor that was diluted in BeneFIX® reconstituted in 10 mM NaCl had a two-phase sedimentation pattern with a clear erythrocyte:plasma interface located 8 mm from the zero-mark and another interface between densely packed and less densely packed erythrocytes located 149 mm from the zero mark.

**Table 6: Erythrocyte Sedimentation of Human Blood Diluted 1:4 in BeneFIX® or 3% Dextran**

| (Sedimentation distance in mm measured from zero-mark) | | | | | | | | | | | |
|---|---|---|---|---|---|---|---|---|---|---|---|
| **Donor & Dilution Time** | **Saline** | **0 mM NaCl** | **10 mM NaCl** | **20 mM NaCl** | **30 mM NaCl** | **40 mM NaCl** | **50 mM NaCl** | **60 mM NaCl** | **77 mM NaCl** | **154 mM NaCl** | **3% Dextran 70** |
| Donor 5 | | | | | | | | | | | |
| 60 min | 2 | 135 | nd | 2 | 2 | 2 | 1 | 1 | 2 | 1 | 132 |
| 90 min | 3 | 145 | nd | 4 | 3 | 3 | 3 | 2 | 2 | 2 | 159 |
| 120 min | 4 | 150 | nd | 5 | 4 | 4 | 4 | 3 | 4 | 3 | 167 |
| 150min | 6 | 153 | nd | 7 | 6 | 5 | 5 | 5 | 5 | 5 | 169 |
| | | | | | | | | | | | |

| Donor 22 | | | | | | | | | | | |
|---|---|---|---|---|---|---|---|---|---|---|---|
| 60 min | 0 | 161 | 4 | 3 | 0 | 0 | 0 | 0 | 0 | nd | 145 |
| 90 min | 3 | 164 | 149 | 3 | 3 | 3 | 2 | 2 | 3 | nd | 166 |
| 120min | 4 | 166 | 151 | 6 | 4 | 4 | 4 | 3 | 4 | nd | 171 |
| 150 min | 7 | 167 | 152 | 8 | 6 | 5 | 5 | 5 | 6 | nd | 172 |

| | | | | | | | | | | | |
|---|---|---|---|---|---|---|---|---|---|---|---|
| NaCl concentrations are those used to reconstitute BeneFIX® | | | | | | | | | | | |

In the second experiment, erythrocyte sedimentation was enhanced by 5% dextrose, "MFR-927" (the formulation buffer for BeneFIX® that lacks rFIX), and BeneFIX® reconstituted with 10 mM NaCl (Table 7). Unlike the first experiment, sedimentation in these tubes was rapid with maximal or nearly maximal sedimentation reached by 15 to 30 minutes.

**Table 7: Erythrocyte Sedimentation of Human Blood Diluted 1:8 in MFR-927, BeneFIX® or 5% Dextrose**

| (Sedimentation distance in mm measured from zero-mark) | | | | | | | | | | |
|---|---|---|---|---|---|---|---|---|---|---|
| **Donor & Dilution Time** | **Saline** | **MFR-927** | **10 mM NaCl** | **25 mM NaCl** | **30 mM NaCl** | **40 mM NaCl** | **50 mM NaCl** | **60 mM NaCl** | **77 mM NaCl** | **5% Dextrose** |
| Donor 56 | | | | | | | | | | |
| 15 min | 0 | >180 | ∼155 | 1 | 0 | 0 | 0 | 0 | 0 | 175 |
| 30 min | 0 | >180 | 177 | 2 | 1 | 0 | 0 | 0 | 0 | 178 |
| 45 min | 1 | >180 | 180 | 3 | 2 | 1 | 1 | 1 | 1 | 179 |
| 60 min | 2 | >180 | >180 | 3 | 2 | 1 | 1 | 1 | 1 | 180 |
| | | | | | | | | | | |

| Donor 57 | | | | | | | | | | |
|---|---|---|---|---|---|---|---|---|---|---|
| 15 min | 0 | >180 | 180 | 1 | 1 | 0 | 0 | 0 | 0 | 179 |
| 30 min | 1 | >180 | >180 | 2 | 2 | 1 | 0 | 1 | 1 | >180 |
| 45 min | 1 | >180 | >180 | 3 | 3 | 1 | 2 | 1 | 1 | >180 |
| 60 min | 2 | >180 | >180 | 4 | 3 | 2 | 3 | 2 | 2 | >180 |

| | | | | | | | | | | |
|---|---|---|---|---|---|---|---|---|---|---|
| NaCl concentrations are those used to reconstitute BeneFIX®; MFR-927 is the formulation buffer for BeneFIX® that lacks rFIX. | | | | | | | | | | |

The results of these experiments demonstrate that the modified Westergren method used to measure erythrocyte sedimentation can be adapted to assess erythrocyte aggregation induced by pharmaceutical preparations or formulations. For example, measurement of erythrocyte sedimentation in Westergren tubes 60 minutes after loading blood diluted 1:4 with test solutions is sufficient to distinguish between agents that enhance aggregation (*i.e.,* 5% dextrose, 3% dextran 70, MFR-927, and BeneFIX® reconstituted in sWFI) from those that do not (*i.e.,* saline and BeneFIX® reconstituted in diluents containing about 25 mM or more NaCl).

### Example 3: Adequacy of 40 mM NaCl in Diluent for Reformulated BeneFIX® to Ameliorate Formulation-Associated Erythrocyte Aggregation

The non-ionic formulation of currently marketed BeneFIX® has been associated with *in vitro* erythrocyte aggregation, which can occur during administration when patient blood is mixed with BeneFIX® in intravenous tubing. The invention provides the discovery that erythrocyte aggregation is associated with the formulation, not recombinant Factor IX, and aggregation can be prevented by reconstituting BeneFIX® with diluents that contain at least 40 mM NaCl.

A goal of this Example is to test the robustness of a 40 mM diluent for reconstituting reformulated BeneFIX® (BeneFIX®-R) preparations. Specifically, experiments were designed to determine whether NaCl concentrations that deviate from 40 mM by as much as 10% are sufficient to prevent formulation-associated erythrocyte aggregation, which was assessed by the adapted, modified Westergren method to measure erythrocyte sedimentation rate (ESR). In addition, the robustness of the NaCl concentration on erythrocyte sedimentation was assessed in both high (*i.e.,* 2000 IU) and low (*i.e.,* 250 IU) dose vials of BeneFIX®-R.

The effect of BeneFIX®-R formulations, MFR-927, and 3% dextran 70 on erythrocyte sedimentation was measured at room temperature using an adaptation of the modified Westergren method, as described in Example 2. The study design is outlined in Table 8. Treatments 1, 2, and 9 are reference treatments.

**Table 8: Study Design for Example 3**

| **Group** | **Treatment** | **Number of Experiments** | **Total Number of Donor Samples Evaluated** | **Dilution of Blood Evaluated** |
|---|---|---|---|---|
| 1 | Saline | 4 | 12 | 1:4 |
| 2 | MFR-927 | 4 | 12 | 1:4 |
| 3 | BeneFIX®-R (55 IU/mL); 36 mM NaCl | 4 | 12 | 1:4 |
| 4 | BeneFIX®-R (55 IU/mL); 40 mM NaCl | 4 | 12 | 1:4 |
| 5 | BeneFIX®-R (55 IU/mL); 44 mM NaCl | 4 | 12 | 1:4 |
| 6 | BeneFIX®-R (440 IU/mL); 36 mM NaCl | 4 | 12 | 1:4 |
| 7 | BeneFIX®-R (440 IU/mL); 40 mM NaCl | 4 | 12 | 1:4 |
| 8 | BeneFIX®-R (440 IU/mL); 44 mM NaCl | 4 | 12 | 1:4 |
| 9 | 3% Dextran 70 | 4 | 12 | 1:4 |

On the day of an experiment, both high (vials of lyophilized BeneFIX®-R containing ∼2000 IU rFIX) and low dosage vials of BeneFIX®-R (vials of lyophilized BeneFIX®-R containing ∼250 IU rFIX) were reconstituted immediately before use with 5 mL of 36 mM, 40 mM, or 44 mM NaCl. The positive control solution 3% (w/v) dextran 70 was prepared in sterile Dulbecco's modified, calcium- and magnesium-free phosphate-buffered saline (PBS-CMF; pH 7.4). Human blood was obtained from donors and collected into Vacutainer tubes containing EDTA. Blood samples were used for erythrocyte sedimentation experiments within 3 hours of collection.

Whole blood was diluted 1:4 in test solutions (*i.e.,* 400 µL of whole blood was added to 1.2 mL of test solution), mixed well, and then loaded into self-zeroing disposable glass Westergren tubes that were held absolutely vertical in a dedicated custom rack. Erythrocyte sedimentation after 60 minutes, which is the distance in mm from the zero mark at the top of the tube to the plasma-erythrocyte interface, was measured and recorded.

Results from all 12 donors were similar. Currently marketed BeneFIX® formulation buffer that lacked rFIX (MFR-927) and 3% dextran 70, a standard positive control solution, displayed enhanced erythrocyte sedimentation as compared to blood that had been mixed in NaCl test solutions (see Figure 1). Regardless of NaCl concentration in the buffer (36 mM, 40 mM, or 44 mM) or rFIX concentration in the product, BeneFIX®-R did not enhance erythrocyte sedimentation as long as it was reconstituted with a NaCl diluent.

The results from this study demonstrate that NaCl concentrations 10% higher or lower than 40 mM in the BeneFIX®-R diluents are sufficient to prevent enhanced erythrocyte sedimentation or aggregation (agglutination). Results also show that the ameliorating effect of NaCl on formulation-associated erythrocyte aggregation was not affected by the concentration of the active ingredient (*i.e.,* rFIX).

### Example 4: Role of Ionic Strength in the Buffer for Reconstituting BeneFIX® in Causing RBC Agglutination

Human blood was collected by venipuncture from four different donors into standard heparinized collecting tubes. Formation of RBC agglutinates was tested by drawing up 2.6 mL of buffer or reconstituted BeneFIX® protein into syringes followed by 0.6 mL of heparinized blood. The mixing/sedimentation behavior of the agglutinates was observed in these syringes over time and photographically recorded using a digital camera.

The composition of the current BeneFIX® formulation pre-lyophilization is rFIX, about 10 mM histidine, about 260 mM glycine, about 1% sucrose, and about 0.005% polysorbate-80, pH 6.8. The BeneFIX® formulation is lyophilized, and prior to injection, reconstituted in sWFI. Originally, the sucrose was suspected to be the cause of agglutination. Hence, test buffers were formulated with 1%, 0.5%, or 0% sucrose. Each sucrose test buffer was drawn up in syringes, followed by a small amount of heparinized blood. The agglutination of RBCs and subsequent rapid sedimentation were found to be identical for the three buffers, suggesting that the sucrose content of the BeneFIX® formulation did not account for the observed agglutination. BeneFIX® reconstituted in normal saline, rather than in sterile water, caused no agglutination. These data indicate that the ionic strength of the BeneFIX® formulation is not sufficient to prevent agglutination when BeneFIX® is reconstituted in water.

In order to determine the minimum ionic strength needed to eliminate agglutination, BeneFIX® was reconstituted (or the pre-lyophilization formulation of BeneFIX® can be varied to include sodium chloride, although methods for lyophilizing sodium chloride formulations is more difficult as compared to lyophilizing formulations that do not have sodium chloride) with solutions having varying concentrations of NaCl (0, 10, 15, 20, 25, 30, 35, 40, 50, 75, 100, and 137 mM). BeneFIX® was also reconstituted in normal saline containing 154 mM NaCl. Only solutions with a sodium chloride concentration from 25 mM to 150 mM are according to the present claims. The other solutions are reference solutions. RBC agglutination and sedimentation behavior was examined in all of these solutions. Table 9 shows the osmolality and ionic strengths (expressed as conductivity) of some of these solutions along with other common intravenous solutions. Upon addition of sodium chloride to the BeneFIX® formulation, whether by straight addition prior to lyophilization or by reconstitution of a lyophilisate with a solution of sodium chloride, the NaCl would be expected to fully dissociate to an equivalent concentration of Na⁺ and Cl⁻ ions. Thus, the ionic strength (expressed in mEq/L (milliequivalents/L) of Na⁺ and Cl⁻) of a formulation buffer plus NaCl would be predicted to be equivalent to the NaCl concentration if the formulation buffer does not contain other ions.

**Table 9: Characterization of Various Solutions**

| **Solution** | **Approximate Calculated Osmolality (mOsm/L)** | **Approximate Calculated Conductivity (mS/cm)** | **pH** |
|---|---|---|---|
| BeneFIX® formulation | 300 | 0 (observed conductivity is <0.2 mS/cm) | 6.8 |
| BeneFIX® formulation + 10 mM NaCl | 320 | 1 | 6.8 |
| BeneFIX® formulation + 20 mM NaCl | 340 | 2 | 6.8 |
| BeneFIX® formulation + 40 mM NaCl | 380 | 4 | 6.8 |
| BeneFIX® formulation + 80 mM NaCl | 460 | 7 | 6.8 |
| BeneFIX® formulation + 137 mM NaCl | 574 | 12.8 | 6.8 |
| BeneFIX® formulation + 154 mM NaCl | 607 | 14.4 | 6.8 |
| 0.9% sodium chloride (equivalent to 154 mM NaCl; 154 mEq/L of Na⁺ and of Cl⁻ ions) | 308 | 14.4 | 6.0 |
| 5% dextrose injection | 250 | 0 | 4.5 |

Upon contact of blood with the solutions in Table 9 in syringes, RBC agglutination was individually observed and then the syringes were gently mixed and inverted to observe blood settling. Agglutinate formation was not observed in all formulations of BeneFIX® reconstituted with at least about 40 mM NaCl (note in the prior Example, about 36 mM NaCl was also sufficient to prevent agglutination). The behavior of blood cells in these solutions containing at least 40 mM NaCl was indistinguishable from that in normal saline.

Blood sedimentation was tested in a series of syringes containing BeneFIX® formulation diluted (if pre-lyophilization)/reconstituted (if lyophilized) in decreasing concentrations of NaCl (starting at 40 mM) 15 minutes after inversion. There was a consistent concentration-dependent response in terms of agglutinate formation and subsequent speed of sedimentation - increasing agglutination and quicker sedimentation with decreasing concentration of NaCl in the buffer. Obvious agglutination was visible with one blood sample in buffer containing ≤25 mM NaCl and with another blood sample in buffer containing ≤30 mM NaCl. In all buffer preparations with ≥40 mM NaCl, the behavior of blood cells was indistinguishable from that in normal saline or BeneFIX® reconstituted in normal saline. BeneFIX® reconstituted with 40 mM NaCl corresponds to a calculated conductivity of 4 mS/cm.

The 5% dextrose injection solution, each mL of which contains 50 mg hydrous dextrose USP in water for injection - has a calculated osmolality of 250 mOsm/L and a calculated ionic strength of 0 mS/cm. This solution, which is commonly administered intravenously, also caused RBC agglutination and very rapid blood sedimentation similar to that observed with BeneFIX® formulation reconstituted in water.

Thus, these results indicate that RBC agglutination caused by BeneFIX® reconstituted in water is due to the BeneFIX® formulation's low ionic strength (0 mEq/L calculated ionic strength, <0.2 mS/cm measured conductivity). This problem can be corrected by reconstituting in NaCl solutions having ≥40 mM NaCl such that the reconstituted solution for injection has a calculated ionic strength of about 40 mEq/L (the sufficient ionic strength to prevent agglutination can also be calculated as a conductivity of about 4 mS/cm or higher) or higher.

## Claims

1. A lyophilized Factor IX formulation and a 25 mM to 150 mM sodium chloride solution for use in a method for preparing the lyophilized Factor IX formulation for intravenous injection wherein the method comprises adding the sodium chloride solution to the lyophilized formulation thereby resulting in a formulation prepared for intravenous injection, wherein the prepared formulation is about isotonic with respect to plasma and has an osmolarity that is from 270 mOsm/L to 330 mOsm/L, or,
is slightly hypotonic with respect to plasma and has an osmolarity that is 220 mOsm/L to 270 mOsm/L, or,
is slightly hypertonic with respect to plasma and has an osmolarity that is 330 mOsm/L to 600 mOsm/L,
and wherein the prepared formulation has a sufficient ionic strength to prevent erythrocyte agglutination upon intravenous injection,
wherein the lyophilized formulation does not contain more than 25mM of an ionizing salt when it is reconstituted in water in a volume that is the same as the fill volume.

2. The lyophilized Factor IX formulation and sodium chloride solution of claim 1 wherein the prepared formulation has an ionic strength of at least 25mEq/L Na⁺ and Cl⁻ ions.

3. The lyophilized Factor IX formulation and sodium chloride solution of any one of claim 1 or 2 wherein the lyophilized formulation does not contain more than 5mM of an ionizing salt when it is reconstituted in water in a volume that is the same as the fill volume.

4. The lyophilized Factor IX formulation and sodium chloride solution of any one of claims 1 to 3 wherein Factor IX is a modified version of Factor IX selected from pegylated factor IX or protein fusion comprising factor IX.

5. The lyophilized Factor IX formulation and sodium chloride solution of claim 4 wherein the protein fusion comprising Factor IX is whole immunoglobulin or domains thereof-Factor IX.

6. The lyophilized Factor IX formulation and sodium chloride solution of any one of claims 1 to 5 wherein the lyophilized formulation comprises a non-ionizing bulking agent.

7. The lyophilized Factor IX formulation and sodium chloride solution of claim 6 wherein the non-ionizing bulking agent is selected from mannitol, glycine, sucrose or lactose.

8. The lyophilized Factor IX formulation and sodium chloride solution of any one of claims 1 to 7 wherein the lyophilized formulation comprises a surfactant.

9. The lyophilized Factor IX formulation and sodium chloride solution of claim 8 wherein the surfactant is selected from polysorbate 80, Triton X-10 and sodium dodecyl sulfate.

10. The lyophilized Factor IX formulation and sodium chloride solution of any one of claims 1 to 9 wherein the lyophilized formulation comprises a sugar or a polyol.

11. The lyophilized Factor IX formulation and sodium chloride solution of claim 10 wherein the sugar or polyol is selected from sucrose, glycerol, xylitol, sorbitol, mannitol, glucose, inositol, raffinose, maltotriose, lactose and trehalose.

12. The lyophilized Factor IX formulation and sodium chloride solution of any one of claims 1 to 11 wherein the lyophilized formulation comprises a buffering agent.

13. The lyophilized Factor IX formulation and sodium chloride solution of claim 12 wherein the buffering agent is selected from acetate, citrate, glycine, histidine, sodium phosphate, potassium phosphate, diethanolamine, and tris.

14. A kit comprising a lyophilized Factor IX formulation and sodium chloride solution according to any one of claims 1 to 13.

## Patentansprüche

1. Lyophilisierte Faktor-IX-Formulierung und 25 mM bis 150 mM Natriumchloridlösung zur Verwendung in einem Verfahren zur Herstellung der lyophilisierten Faktor-IX-Formulierung zur intravenösen Injektion, wobei das Verfahren umfasst: das Zugeben der Natriumchloridlösung zu der lyophilisierten Formulierung, was eine zur intravenösen Injektion hergestellte Formulierung ergibt, wobei die hergestellte Formulierung etwa isotonisch bezüglich des Plasmas ist und eine Osmolarität aufweist, welche 270 mOsm/l bis 330 mOsm/l beträgt, oder
leicht hypotonisch bezüglich des Plasmas ist und eine Osmolarität aufweist, welche 220 mOsm/l bis 270 mOsm/l beträgt, oder leicht hypertonisch bezüglich des Plasmas ist und eine Osmolarität aufweist, welche 330 mOsm/l bis 600 mOsm/l beträgt, und wobei die hergestellte Formulierung eine ausreichende Ionenstärke aufweist, um Erythrozytenagglutination bei intravenöser Injektion zu verhindern,
wobei die lyophilisierte Formulierung nicht mehr als 25 mM eines ionisierenden Salzes enthält, wenn sie in Wasser rekonstituiert wird in einem Volumen, das das Gleiche wie das Füllvolumen ist.

2. Lyophilisierte Faktor-IX-Formulierung und Natriumchloridlösung nach Anspruch 1, wobei die hergestellte Formulierung eine Ionenstärke von mindestens 25 mEq/l Na⁺- und Cl⁻-Ionen aufweist.

3. Lyophilisierte Faktor-IX-Formulierung und Natriumchloridlösung nach einem der Ansprüche 1 oder 2, wobei die lyophilisierte Formulierung nicht mehr als 5 mM eines ionisierenden Salzes enthält, wenn sie in Wasser in einem Volumen rekonstituiert wird, das das Gleiche wie das Füllvolumen ist.

4. Lyophilisierte Faktor-IX-Formulierung und Natriumchloridlösung nach einem der Ansprüche 1 bis 3, wobei der Faktor IX eine modifizierte Version von Faktor IX, ausgewählt aus pegyliertem Faktor IX oder Protein-Fusion, umfassend Faktor IX, ist.

5. Lyophilisierte Faktor-IX-Formulierung und Natriumchloridlösung nach Anspruch 4, wobei die Protein-Fusion, umfassend Faktor IX, Gesamtimmunoglobulin- oder -Domänen davon -Faktor-IX ist.

6. Lyophilisierte Faktor-IX-Formulierung und Natriumchloridlösung nach einem der Ansprüche 1 bis 5, wobei die lyophilisierte Formulierung einen nichtionisierenden Füllstoff enthält.

7. Lyophilisierte Faktor-IX-Formulierung und Natriumchloridlösung nach Anspruch 6, wobei der nichtionisierende Füllstoff ausgewählt ist aus Mannitol, Glycin, Sucrose oder Lactose.

8. Lyophilisierte Faktor-IX-Formulierung und Natriumchloridlösung nach einem der Ansprüche 1 bis 7, wobei die lyophilisierte Formulierung ein Surfactant umfasst.

9. Lyophilisierte Faktor-IX-Formulierung und Natriumchloridlösung nach Anspruch 8, wobei das Surfactant ausgewählt ist aus Polysorbat 80, Triton X-10 und Natriumdodecylsulfat.

10. Lyophilisierte Faktor-IX-Formulierung und Natriumchloridlösung nach einem der Ansprüche 1 bis 9, wobei die lyophilisierte Formulierung einen Zucker oder ein Polyol umfasst.

11. Lyophilisierte Faktor-IX-Formulierung und Natriumchloridlösung nach Anspruch 10, wobei der Zucker oder das Polyol ausgewählt ist aus Sucrose, Glycerol, Xylitol, Sorbitol, Mannitol, Glucose, Inosit, Raffinose, Maltotriose, Lactose und Trehalose.

12. Lyophilisierte Faktor-IX-Formulierung und Natriumchloridlösung nach einem der Ansprüche 1 bis 11 wobei die lyophilisierte Formulierung ein Pufferungsmittel umfasst.

13. Lyophilisierte Faktor-IX-Formulierung und Natriumchloridlösung nach Anspruch 12, wobei das Pufferungsmittel ausgewählt ist aus Acetat, Citrat, Glycin, Histidin, Natriumphosphat, Kaliumphosphat, Diethanolamin und Tris.

14. Kit, umfassend eine lyophilisierte Faktor-IX-Formulierung und Natriumchloridlösung nach einem der Ansprüche 1 bis 13.

## Revendications

1. Formulation de facteur IX lyophilisée et solution de chlorure de sodium 25 mM à 150 mM pour une utilisation dans un procédé de préparation de la formulation de facteur IX lyophilisée pour une injection intraveineuse, le procédé comprenant l'ajout de la solution de chlorure de sodium à la formulation lyophilisée résultant ainsi en une formulation préparée pour une injection intraveineuse, la formulation préparée étant à peu près isotonique par rapport au plasma et a une osmolarité qui est de 270 mOsm/l à 330 mOsm/l, ou,
est légèrement hypotonique par rapport au plasma et a une osmolarité qui est de 220 mOsm/l à 270 mOsm/l, ou,
est légèrement hypertonique par rapport au plasma et a une osmolarité qui est de 330 mOsm/l à 600 mOsm/l,
et la formulation préparée ayant une force ionique suffisante pour empêcher l'agglutination des érythrocytes lors d'une injection intraveineuse,
la formulation lyophilisée ne contenant pas plus de 25 mM d'un sel ionisant lorsqu'elle est reconstituée dans l'eau en un volume qui est le même que le volume de remplissage.

2. Formulation de facteur IX lyophilisée et solution de chlorure de sodium selon la revendication 1, la formulation préparée ayant une force ionique d'au moins 25 mEq/l d'ions Na⁺ et Cl⁻.

3. Formulation de facteur IX lyophilisée et solution de chlorure de sodium selon l'une quelconque des revendications 1 ou 2, la formulation lyophilisée ne contenant pas plus de 5 mM d'un sel ionisant lorsqu'elle est reconstituée dans l'eau en un volume qui est le même que le volume de remplissage.

4. Formulation de facteur IX lyophilisée et solution de chlorure de sodium selon l'une quelconque des revendications 1 à 3, le facteur IX étant une version modifiée du facteur IX sélectionnée parmi le facteur IX pégylé ou une fusion protéique comprenant le facteur IX.

5. Formulation de facteur IX lyophilisée et solution de chlorure de sodium selon la revendication 4, la fusion protéique comprenant le facteur IX étant l'immunoglobuline totale ou des domaines de celle-ci-facteur IX.

6. Formulation de facteur IX lyophilisée et solution de chlorure de sodium selon l'une quelconque des revendications 1 à 5, la formulation lyophilisée comprenant un diluant non ionisant.

7. Formulation de facteur IX lyophilisée et solution de chlorure de sodium selon la revendication 6, le diluant non ionisant étant sélectionné parmi le mannitol, la glycine, le saccharose ou le lactose.

8. Formulation de facteur IX lyophilisée et solution de chlorure de sodium selon l'une quelconque des revendications 1 à 7, la formulation lyophilisée comprenant un tensioactif.

9. Formulation de facteur IX lyophilisée et solution de chlorure de sodium selon la revendication 8, le surfactant étant sélectionné parmi le polysorbates 80, le triton X-10 et le dodécyl sulfate de sodium.

10. Formulation de facteur IX lyophilisée et solution de chlorure de sodium selon l'une quelconque des revendications 1 à 9, la formulation lyophilisée comprenant un sucre ou un polyol.

11. Formulation de facteur IX lyophilisée et solution de chlorure de sodium selon la revendication 10, le sucre ou le polyol étant sélectionné parmi le saccharose, le glycérol, le xylitol, le sorbitol, le mannitol, le glucose, l'inositol, le raffinose, le maltotriose, le lactose et le tréhalose.

12. Formulation de facteur IX lyophilisée et solution de chlorure de sodium selon l'une quelconque des revendications 1 à 11, la formulation lyophilisée comprenant un agent tampon.

13. Formulation de facteur IX lyophilisée et solution de chlorure de sodium selon la revendication 12, l'agent tampon étant sélectionné parmi l'acétate, le citrate, la glycine, l'histidine, le phosphate de sodium, le phosphate de potassium, la diéthanolamine, et le tris.

14. Kit comprenant une formulation de facteur IX lyophilisée et une solution de chlorure de sodium selon l'une quelconque des revendications 1 à 13.
